# EUROPEAN PATENT SPECIFICATION

(11) **EP 3 729 078 B1**
(45) Date of publication and mention of the grant of the patent: **21.08.2024**
(21) Application number: 18822042.0
(22) Date of filing: 17.12.2018
(51) Int. Cl.: G01N 33/49

(54) **IN-VITRO HEMOLYSIS DETECTION AND CORRECTION OF AT LEAST ONE BLOOD PARAMETER IN A WHOLE BLOOD SAMPLE**
IN-VITRO-HÄMOLYSENACHWEIS UND KORREKTUR MINDESTENS EINES BLUTPARAMETERS IN EINER VOLLBLUTPROBE
DÉTECTION D'HÉMOLYSE IN VITRO ET CORRECTION D'AU MOINS UN PARAMÈTRE SANGUIN DANS UN ÉCHANTILLON DE SANG TOTAL

(30) Priority: 20.12.2017 DK PA201700728
(43) Date of publication of application: 28.10.2020
(73) Proprietor: Radiometer Medical ApS, 2700 Brønshøj (DK)
(72) Inventor: KJÆR, Thomas, 2700 Brønshøj (DK); NEWLOVE, Pauline, 2700 Brønshøj (DK); CLAUSEN, Lydia, Dahl, 2700 Brønshøj (DK); LAGONI, Signe, 2700 Brønshøj (DK)
(74) Representative: Inspicos P/S
(86) International application number: PCT/EP2018/085124
(87) International publication number: WO 2019/121459

(56) References cited:
- VERMEER HENRICUS J ET AL: "CORRECTION OF PATIENT RESULTS FOR BECKMAN COULTER LX-20 ASSAYS AFFECTED BY INTERFERENCE DUE TO HEMOGLOBIN, BILIRUBIN OR LIPIDS: A PRACTICAL APPROACH", CLINICAL CHEMISTRY AND LABORATORY MEDI, DE GRUYTER, DE, vol. 45, no. 1, 1 January 2007 (2007-01-01), pages 114 - 119, XP008076581, ISSN: 1434-6621, DOI: 10.1515/CCLM.2007.004
- "In Vitro and In Vivo Hemolysis - An Unresolved Dispute in Laboratory Medicine", 31 December 2012, DE GRUYTER, ISBN: 978-3-11-024613-1, ISSN: 2305-2198, article G LIPPI ET AL: "How to distinguish in vivo versus in vitro hemolysis", pages: 35 - 37, XP055574287

## Description

### Technical field

The present invention relates to the field of diagnostic blood sample analysis.

### Background of the invention

Hemolysis is a frequently encountered phenomenon in whole blood, serum, and plasma samples. When hemolysis has occurred in a blood sample, it may affect the measurement of a number of different blood parameters compared to non-hemolysed blood samples because the concentration of analytes in whole blood may differ significantly from the concentration of analytes found within the red blood cells. For example, in whole blood plasma, potassium levels are usually about 4.0 mM, while the potassium concentration within red blood cells is usually about 150 mM.

Since the concentration of potassium within red blood cells is 25-75 times higher than the concentration of potassium in normal plasma, measuring potassium in the fluid portion of a patient's hemolyzed blood sample will result in a measured value that is increased compared to the potassium level in a non-hemolyzed blood sample. Further, the hemolysis might already have occurred *in vivo* but it may also have occurred *in vitro,* which would have introduced an artifact to the measurement. The potassium concentration in the fluid portion of non-hemolyzed blood is an important indicator of numerous conditions. An over-estimate of the concentration of potassium in hemolyzed blood may for example result in a treatment of the patient for hyperkalemia (increased blood potassium) when the patient may truly have a normal or low concentration of potassium. Already a relatively small number of ruptured red blood cells can result in an artificially elevated blood potassium level caused by hemolysis.

In addition to an elevated plasma potassium concentration, other analytes such as calcium, lactate dehydrogenase, acid phosphatase, aspartate aminotransferase, and alanine aminotransferase, are also present in higher concentration in red blood cells than in the plasma, and these analytes may be artificially elevated due to *in-vitro* hemolysis.

A list of blood parameters that may be affected by hemolysis can for example be found in the publication of Lippi et al., Clin Chem Lab Med 2008;46(6):764-772.

The potentially affected blood parameters are summarized in Table 1.

**Table 1. Blood parameters affected by hemolysis and/or blood cell lysis in the specimen (adapted from Lippi et al.).**

| Parameter | Bias | Cause |
|---|---|---|
| Adrenocorticotropic hormone | Negative | Proteolysis |
| Activated partial thromboplastin time | Negative | Release of thromboplastic |
| | | substances |
| Antithrombin | Negative | Analytical interference |
| Aspartate aminotransferase | Positive | Cellular release |
| Alanine aminotransferase | Positive | Cellular release |
| Albumin | Negative | Dilution |
| Alkaline phosphatase | Negative | Analytical interference |
| Bilirubin (neonatal) | Variable | Analytical interference |
| Bilirubin (total) | Negative | Analytical interference |
| Calcium | Negative | Dilution, protein binding |
| Calcitonine | Positive | Proteolysis |
| Chloride | Negative | Dilution |
| Cortisol | Negative | Analytical interference |
| Creatine kinase | Positive | Analytical interference |
| Creatinine | Positive | Analytical interference |
| D-dimer | Positive | Release of thromboplastic |
| | | substances |
| Fibrinogen | Negative | Release of thromboplastic |
| | | substances |
| Folate | Positive | Cellular release |
| γ-Glutamyltransferase | Negative | Analytical interference |
| Gastrin | Negative | Proteolysis |
| Glucagon | Negative | Proteolysis |
| Glucose | Negative | Dilution |
| Haptoglobin | Negative | Analytical interference |
| Homocysteine | Negative | Analytical interference |
| Insulin | Negative | Proteolysis |
| Iron | Positive | Analytical interference |
| Lactate dehydrogenase | Positive | Cellular release |
| Lipase | Positive | Analytical interference |
| Magnesium | Positive | Cellular release |
| Parathormon | Negative | Proteoloysis |
| Phosphorus | Positive | Cellular release |
| Potassium | Positive | Cellular release |
| Prostate specific antigen | Positive | Analytical interference |
| Prothrombin time | Positive | Release of thromboplastic |
| | | substances |
| Partial O₂ pressure | | |
| Partial CO₂ pressure | | |
| Sodium | Negative | Dilution |
| Urea | Positive | Cellular release |
| Testosterone | Negative | Analytical interference |
| Troponin I | Positive | Analytical interference |
| Troponin T | Negative | Analytical interference |
| Vitamin B12 | Negative | Analytical interference |

EP 0 268 025 B1 describes a method of analysis which makes it possible to measure components in a blood serum sample or a blood plasma sample of hemolyzed blood by reducing errors of measured values.

US 2014/0262831 A1 describes a whole blood hemolysis sensor comprising an electrochemical sensor comprising an oxidoreductase enzyme capable of generating hydrogen peroxide.

EP 2 788 755 B1 describes a visual detection device to detect hemolysis in a whole blood sample from a pierceable container.

WO 2016/054030 A1 describes devices, systems and methods that enable the detection of hemolysis in a sample such that a sample which exhibits an unacceptable level of hemolysis can be immediately flagged or disregarded in an associated diagnostic test.

WO 2017/085180 A1 describes a sensor for the optical detection of free hemoglobin (Hb) in a whole blood sample and a method of analyzing a whole blood sample, wherein the method comprises optically probing a free hemoglobin level of the whole blood sample and measuring on the same whole blood sample a further component present in the whole blood sample; and correcting flagging or discarding the measurement of the further component on the basis of the hemolysis level of the whole blood sample.

VERMEER Henricus J., Correction of patient results for Beckman Coulter LX-20 assays affected by interference due to hemoglobin, bilirubin or lipids: a practical approach, Clin Chem Lab Med 2007;45(1):114-119 describes that the influence of interference by hemolysis, icterus and lipemia on the results of routine chemistries may lead to wrong interpretations and that on Synchron LX-20 instruments (Beckman Coulter) serum or plasma indices can be used as reliable semi-quantitative measures of the magnitude of such interference.

In the light of the above, there is however still a need for improved methods and blood parameter analyzers to differentiate between *in vivo* and *in vitro* hemolysis and to determine the level of *in vitro* hemolysis in whole blood samples. There is further a need to improve the correction of the measured value of at least one blood parameter, such as potassium, based on the hemolysis of the sample.

It is thus an object of the present invention to provide methods for determining and characterizing hemolysis in a whole blood sample. It is a further object of the present invention to correct the measured value of at least one additional blood parameter, such as potassium, based on the hemolysis. Thus, it is an object of the present invention to provide methods for accurately analyzing said at least one blood parameter.

### Summary

The objects are solved by an *in vitro* method according to a first aspect for analyzing at least one blood parameter in a whole blood sample of a patient comprising:
a) determining the *in vivo* hemolysis in a fresh whole blood sample of the patient by immediately performing the hemolysis determination after the blood sample has been obtained from the patient;
b) determining the overall hemolysis and the at least one blood parameter in a whole blood sample of the patient; and
   characterized in that the method is further comprising
c) calculating the *in vitro* hemolysis based on the overall hemolysis and the *in vivo* hemolysis.

The objects are further solved by an analysis apparatus, according to a second aspect, comprising:
- an input unit; and
- a processing unit.

The input unit is configured to receive (i) *in vivo* hemolysis data of a fresh whole blood sample of a patient determined by immediately performing the hemolysis determination after the blood sample has been obtained from the patient; and (ii) overall hemolysis data and data of at least one blood parameter in a whole blood sample of the patient.

The processing unit is configured to calculate the *in vitro* hemolysis data based on the overall hemolysis data and the *in vivo* hemolysis data.

Moreover, according to a third aspect, the present invention relates to a system for analysing a whole blood sample, comprising
- an analysis device according to the second aspect; and
- a remote measurement unit configured to determine *in vivo* hemolysis in a fresh blood sample, and to transmit the *in vivo* hemolysis data to the analysis device.

The input unit of the analysis device is configured to receive the *in vivo* hemolysis data determined and transmitted by the remote measurement unit.

According to a fourth aspect, the present invention relates to a computer program element for controlling an analysis apparatus of the present invention, which, when executed by a processing unit, cause said analysis apparatus to carry out the steps of the method of the first aspect.

According to a fifth aspect, the present invention relates to a computer readable medium having stored the computer program element of the fourth aspect.

### Brief description of the drawings

Figure 1 schematically illustrates an example of a method in accordance with the first aspect.
Figure 2 schematically illustrates an example of an analysis apparatus according to the second aspect.
Figure 3 illustrates a remote measurement device.
Figure 4 schematically illustrates an example of a system in accordance with the third aspect.

### Detailed description

A first aspect of the present invention relates to a method for analyzing at least one blood parameter in a whole blood sample of a patient comprising:
a) determining 10 the *in vivo* hemolysis in a fresh whole blood sample of the patient;
b) determining 12 the overall hemolysis and the at least one blood parameter in a whole blood sample of the patient that is suitable for determining the at least one blood parameter; and
c) calculating 14 the *in vitro* hemolysis based on the overall hemolysis and the *in vivo* hemolysis.

By determining the *in vitro* hemolysis based on the overall hemolysis and the *in vivo* hemolysis, the inventive method provides a means to differentiate between the amount of *in vivo* and *in vitro* hemolysis. This may be valuable information for a physician when interpreting the results of the blood sample and its relevance for the patient.

It is preferred that the inventive method is an *in vitro* method.

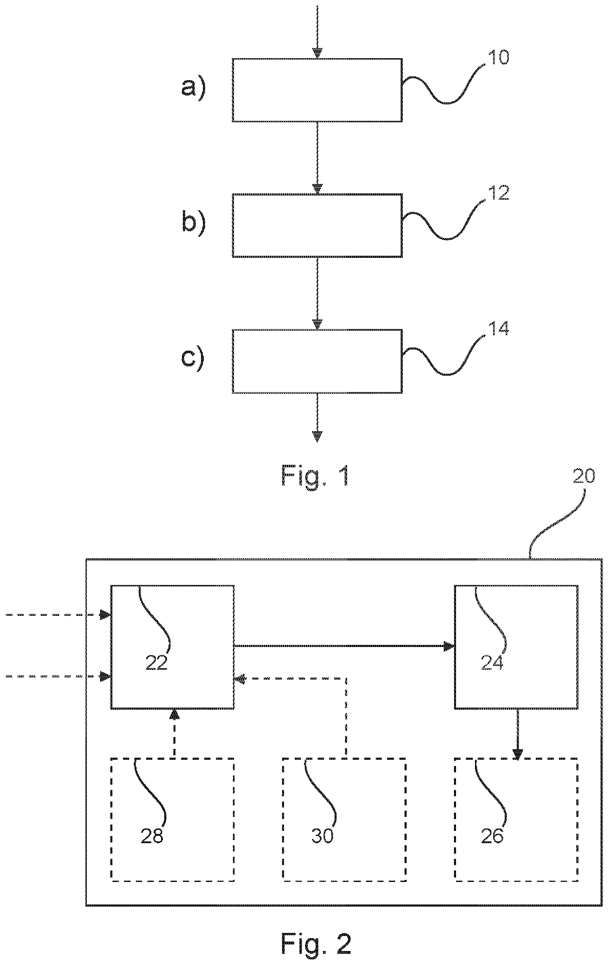

In its broadest sense, the present invention covers all methods comprising the determination of the *in vivo* hemolysis under a) and the determination of the overall hemolysis under b), independent of their order. Thus, in one embodiment, the *in vivo* hemolysis may be determined first and the overall hemolysis is determined afterwards. Figure 1 illustrates such a method in accordance with the first aspect.

In another embodiment, the overall hemolysis is determined first and the *in vivo* hemolysis is determined afterwards. In this embodiment, it is preferred that the *in vivo* hemolysis is only determined in case an overall hemolysis has been detected in the whole blood sample of the patient that is suitable for determining the at least one blood parameter.

The determination of the *in vivo* hemolysis in a fresh whole blood sample may be performed by using commonly known *in vivo* hemolysis assays. In general, the *in vivo* hemolysis may be determined when conditions can be achieved in a blood sample for which the occurrence of an *in vitro* hemolysis is minimized or even completely excluded. According to the present invention, the occurrence of an *in vitro* hemolysis may be minimized by immediately performing the hemolysis determination after the blood sample has been obtained from the patient so that no *in vitro* hemolysis is induced e.g. by specimen transport or delayed processing etc. Further, the risk of an *in vitro* hemolysis can further be minimized by avoiding centrifugation or rigorous mixing of the whole blood sample.

In one embodiment, the determination of the *in vivo* hemolysis is performed using a standalone device, preferably a point of care device. Non-limiting examples of suitable devices for determining the *in vivo* hemolysis include chromatography-based devices or porous mirror devices.

In a preferred embodiment, the *in vivo* hemolysis is determined by using a porous mirror device. The device is typically configured to comprise pores in a translucent sampler which are not accessible for red blood cells but free hemoglobin may enter the pores and the concentration thereof may then be determined by optical detection of the free hemoglobin. Such porous mirror devices are for example described in WO 2017/085180 A1 and WO 2017/085162 A1. The devices may comprise a sensor for the optical detection of free hemoglobin in a whole blood sample, the sensor comprising a translucent slab with a front side and a back side facing away from the front side, wherein the front side is adapted for being contacted with a whole blood sample; a reflective layer at the front side of the translucent slab, the reflective layer being adapted to reflect light reaching the reflective layer from the translucent slab; an optical probing device comprising a light source and a detector, wherein the light source is adapted to illuminate at least pores in the translucent slab, wherein the detector is arranged to receive light emerging from the pores in response to an illumination by the light source, and wherein the detector is adapted to generate a signal representative of the detected light. The translucent slab is provided with dead-end pores extending from the front side into the translucent slab in a direction towards the backside. Each of the pores has a respective opening in the front side of the translucent slab penetrating the reflecting layer. A cross-sectional dimension of the openings of the pores is dimensioned so as to prevent red blood cells from entering the pores, while allowing free hemoglobin to enter the pores.

In another embodiment, the *in vivo* hemolysis may be determined using a chromatographic assay device. Such a chromatographic detection device contains a sample application site and a detection site. Fluid comprising free hemoglobin, but not the red blood cells, may travel from the application site due to capillary forces and be detected at the detection site, e.g. by optical detection. Non-limiting examples of such a chromatographic detection device are described in WO 2015/191450 A1.

In one embodiment, the chromatographic device for determining the *in vivo* hemolysis is based on using a filter paper as chromatographic material.

In another embodiment, the *in vivo* hemolysis may be determined using a visual detection device as described in EP 2 788 755 B1 (see e.g. claim 1). A semi-quantitative device is for example commercially available as Helge^{™} (Hemcheck Sweden AB).

Other methods for determining the *in vivo* hemolysis in a fresh blood sample may comprise determining the level of methemoglobin. Such methods are for example described in WO 2016/054033 A1, wherein after measuring a first amount of methemolgobin, the sample is contacted with an oxidant to oxidize free hemoglobin and measuring a second amount of methemoglobin.

Typically, the determination of the overall hemolysis and the at least one blood parameter in a whole blood sample of the patient that is suitable for determining the at least one blood parameter is performed on the same analyzer but may also be performed using separate analyzers. In a preferred embodiment, the determination of the overall hemolysis and/or the at least one blood parameter is performed using a blood gas analyzer.

The whole blood sample used for the determination of the overall hemolysis and the at least one blood parameter is typically at risk that an *in vitro* hemolysis might have occurred. The analyzer is typically not present at the site where the whole blood sample had been obtained from the patient and thus needs to be transported to the analyzer. Further, samples for blood gas analysis need to be capped and/or the air needs to be removed and the sample is mixed with an anticoagulant. During all these steps, an *in vitro* hemolysis may occur.

In a preferred embodiment, the *in vivo* hemolysis is determined in a fresh blood sample obtained by a finger prick, wherein preferably the first three blood drops are not used for the determination of the *in vivo* hemolysis.

In another embodiment, the fresh whole blood sample for the *in vivo* hemolysis determination was obtained by drawing a syringe.

In a preferred embodiment, the fresh whole blood sample of the patient and the whole blood sample of the patient that is suitable for determining the at least one blood parameter were obtained at essentially the same time. In a preferred embodiment, the two blood samples were obtained within 100 minutes, preferably within 10 minutes, more preferably within 5 minutes and even more preferably within 3 minutes.

In another preferred embodiment, the sample for determining the overall hemolysis and the at least one blood parameter in a whole blood sample of the patient that is suitable for determining the at least one blood parameter is drawn no more than 15 minutes before the fresh whole blood sample has been obtained from the patient, preferably no more than 5 minutes before the fresh whole blood sample has been obtained from the patient.

In another preferred embodiment, the sample for determining the overall hemolysis and the at least one blood parameter in a whole blood sample of the patient that is suitable for determining the at least one blood parameter is drawn no more than 60 minutes after the fresh whole blood sample has been obtained from the patient, preferably no more than 30 minutes after the fresh whole blood sample has been obtained from the patient, and even more preferably no more than 10 minutes after the fresh whole blood sample has been obtained from the patient, and even more preferably no more than 5 minutes after the fresh whole blood sample has been obtained from the patient.

In another preferred embodiment, the fresh whole blood sample and the whole blood sample of the patient that is suitable for determining the at least one blood parameter are the same blood sample which is however measured at different time points, e.g. wherein the *in vivo* hemolysis is determined immediately, e.g. within 10 minutes, after the blood sample was obtained and the overall hemolysis and the at least one blood parameter are measured in the whole blood sample after further processing the sample, e.g. after attaching the cap and ventilating the sampler comprising the whole blood sample, dissolving an anticoagulant, such as heparin, in the whole blood sample, transporting the sample to the analyzer and/or mixing the sample. In a particularly preferred embodiment, the whole blood sample is provided in a porous mirror blood sampler, wherein the *in vivo* hemolysis may be determined optically before further processing the whole blood sample and wherein the porous mirror blood sampler is suitable for determining the overall hemolysis and the at least one additional one blood parameter, preferably in a blood gas analyzer.

In one embodiment, if an *in vitro* hemolysis is detected after calculating the *in vitro* hemolysis based on the overall hemolysis and the *in vivo* hemolysis, the whole blood sample of the patient that is suitable for determining the at least one blood parameter is flagged. Due to the presence of the *in vivo* and the *in vitro* hemolysis, a physician needs to decide how to interpret the *in vivo* and *in vitro* hemolysis results and whether the hemolysis may potentially have affected the measurement of the at least one blood parameter.

In a preferred embodiment, the calculation of the *in vitro* hemolysis comprises performing a calculation comprising subtracting the value for the *in vivo* hemolysis from the overall hemolysis. This calculation may however include the use of additional conversion factors, e.g. if the *in vivo* hemolysis is determined semi-quantitatively, e.g. as "low", "medium" or "high", or if the *in vivo* hemolysis is measured in a different unit as the overall hemolysis.

In one embodiment, the calculation of the *in vitro* hemolysis may comprise performing a calculation based on an averaged *in vivo* hemolysis value, in case the *in vivo* hemolysis has been obtained in several different blood samples from the same patient and the results may be averaged as e.g. a mean *in vivo* hemolysis.

In a preferred embodiment, the inventive method further comprises d) correcting the value for at least one parameter as determined in step b) based on the calculated *in vitro* hemolysis.

The correction of the value for the at least one parameter may occur in addition or instead of flagging the whole blood sample as described above. The correction of the value of the at least one parameter based on the *in vitro* hemolysis instead of the overall hemolysis provides a more accurate corrected test result than a correction being merely based on the overall hemolysis.

In a preferred embodiment, step d) comprises performing a calculation comprising subtracting a factor based on the *in vitro* hemolysis from the value of at least one additional blood parameter. This calculation typically also requires the use of a conversion factor for converting the degree of *in vitro* hemolysis into corresponding units of the at least one blood parameter. The conversion factor, which may also be called α factor, may be determined experimentally, e.g. by using a series of samples with a known amount of hemolysis, e.g. by mixing a non-hemolysed sample with a completely hemolysed blood sample to introduce a known amount of e.g. free hemoglobin in the non-hemolysed sample, and then determine the at least one blood parameter in the series of samples. The data (blood parameter over known concentration of free hemoglobin) for the series of samples may then be fitted, e.g. by using linear regression and a conversion factor may be determined. For example, the conversion factor for potassium as the at least one blood parameter is -3 µM K⁺/(mg Hb/dL). The conversion factor for calcium is 0.13 µM Ca²⁺/(mg Hb/dL). (see Harmonization in Hemolysis detection and prevention. A working Group of the Catalonian Health Institute (ICS) experience. Fernandez, P. et al., Clin. Chem. Lab. Med. 2014, 52(11), p. 1557.) Conversion factors for other blood parameters may easily be determined by the person skilled in the art accordingly.

In yet another preferred embodiment, the at least one blood parameter is at least one of the blood parameters of Table 1.

In a preferred embodiment, the at least one blood parameter is selected from the group consisting of aspartate aminotransferase, alanine aminotransferase, creatinine, creatine kinase, iron, lactate dehydrogenase, lipase, magnesium, phosphorus, potassium, calcium, partial pressure of oxygen, partial pressure of carbon dioxide, and urea.

In another preferred embodiment, the at least one blood parameter is selected from the group consisting of potassium, calcium, lactate dehydrogenase, partial pressure of oxygen, partial pressure of carbon dioxide, and sodium.

In yet another preferred embodiment, the at least one blood parameter is selected from the group consisting of potassium, calcium, lactate dehydrogenase and sodium.

In yet another preferred embodiment, the at least one blood parameter is potassium, calcium and/or sodium.

In a particularly preferred embodiment, the at least one blood parameter is potassium. For potassium, in a preferred embodiment, a correction of the at least one blood parameter based on the *in vitro* hemolysis value can be performed for an *in vitro* hemolysis value of up to 1000 mg Hb/dL, preferably to a value of 300 mg Hb/dL, or to a value of up to 200 mg Hb/dL. Normally, blood samples that show a hemolysis of up to50- 100 mg Hb/dL are normally used without any correction according to the state of the art. (see Harmonization in Hemolysis detection and prevention. A working Group of the Catalonian Health Institute (ICS) experience. Fernandez, P. et al., Clin. Chem. Lab. Med. 2014, 52(11), p. 1557.)

In another preferred embodiment, the at least one blood parameter is calcium.

According to a second aspect, the present invention also relates to an analysis apparatus 20 comprising:
- an input unit 22; and
- a processing unit 24.

The input unit is configured to receive (i) *in vivo* hemolysis data of a fresh whole blood sample of a patient, and (ii) overall hemolysis data, and optionally data of at least one blood parameter, in a whole blood sample of the patient that is suitable for determining the at least one blood parameter.

The processing unit is configured to calculate the *in vitro* hemolysis data based on the overall hemolysis data and the *in vivo* hemolysis data.

Figure 2 schematically illustrates an example of an analysis apparatus 20 in accordance with the second aspect.

In the example of figure 2, the analysis apparatus 20 is implemented as a data processor, for example as a personal computer (PC), personal digital assistant (PDA), a smartphone, or as a custom-designed item of electronic equipment.

The input unit 22 is capable of receiving input data from a wide variety of sources. For example, the input unit 22 may comprise a keyboard interface to enable a user manually to enter measured *in vivo* hemolysis data, overall hemolysis data, and optionally blood parameter data using a physical keyboard, or a keyboard presented via an on-screen, or touch-screen interface.

Optionally, input unit 22 comprises a wired data interface in accordance with, for example, the Universal Serial Bus (USB) standards. Optionally, input unit 22 comprises a Local Area Network (LAN) and/or a Wide Area Network interface to enable measured *in vivo* hemolysis data, overall hemolysis data, and optionally blood parameter data to be obtained from a Local Area Network or a more remote location.

Optionally, input unit 22 comprises a wireless data interface in accordance with, for example, the Bluetooth (TM) and/or the IEEE 802.11 Wireless Local Area Network, and/or the GSM, CDMA, LTE mobile telephone standards to enable measured *in vivo* hemolysis data, overall hemolysis data, and optionally blood parameter data to be provided to the analysis apparatus from a remote reader located remote from the analysis apparatus.

Optionally, input unit 22 comprises an optical data and/or infrared (IR) interface enabling measured *in vivo* hemolysis data, overall hemolysis data, and optionally blood parameter data to provide to the analysis apparatus by aligning a handheld unit comprising an optical data and/or IR transmitter with the optical data and/or infrared interface of the analysis apparatus.

The skilled person will appreciate that a wide variety of techniques for inputting data into the analysis apparatus 20 are available.

Preferably, input unit 22 is configured to receive measured *in vivo* hemolysis data, overall hemolysis data, and optionally blood parameter data transmitted digitally from a device that has been used to obtain the in vivo hemolysis data and the device used for determining the overall hemolysis and the at least one additional blood parameter, such as a point-of-care device.

The processing unit 24 is operably connected to the input unit 22 so that measured *in vivo* hemolysis data, overall hemolysis data, and optionally blood parameter data are presented to the processing unit 24 to enable the calculation of *in vitro* analysis data according to the method of the first aspect.

Optionally, the processing unit 24 is provided as the central processing unit (CPU) of a personal computer (PC), or a personal digital assistant (PDA). Alternatively, the processing unit 24 is provided as a smartphone processor. Alternatively, a custom-designed analysis apparatus 20 may be provided, in which case the processing unit 24 may be a microprocessor (such as an ARM Cortex TM series processor), a microcontroller (such as a microcontroller from the Intel TM 80296 family), a digital signal processor (DSP), or a field programmable gate array (FPGA).

Of course, the skilled person will appreciate that there are many variations in the implementation of a suitable processing unit 24, and that ancillary components such as a Basic Input Output System (BIOS), a random access memory (RAM), a read-only memory (ROM), power supply and conditioning circuitry, communications buses, can optionally be implemented dependent on the particular use-case.

Furthermore, a skilled person will appreciate that although a processing unit 24 may be provided which exclusively performs steps according to the method of the first aspect or its optional embodiments, this is not essential. Optionally a processing unit 24 can be provided which performs other operations, such as the execution of an operating system and other application software, in addition to the steps according to the method of first aspect and its optional embodiments.

In operation, an electrical supply is applied to the analysis apparatus 20. The processing unit 24 and the input unit 22 perform their standard initialisation operations. The processing unit 24 and the input unit 22 then wait for a user of the analysis apparatus 20 provide input data, via manual keyboard entry, or wired, wireless or optical means, as generally described above. When the input unit 22 receives the *in vivo* hemolysis data and overall hemolysis data, it transmits it via a data bus of the analysis apparatus 20 to the processing unit 24. Upon reception of the *in vivo* hemolysis data and overall hemolysis data, computer processing instructions comprising a hemolysis analysis subroutine in accordance with the method of the first aspect, or its optional embodiments, are loaded into the processing unit 24. The processing unit 24 performs, for example one or a combination of arithmetic operations, look-up table operations, curve-fitting operations or equivalents, as defined by the hemolysis analysis subroutine. The output of the hemolysis analysis subroutine is *in vitro* hemolysis data based on the input data.

Optionally, the processing unit 24 stores the result of the calculation (the *in vitro* hemolysis data) in random access memory. Optionally, the processing unit 24 transmits the result of the calculation to a display means or a communication means.

In a preferred embodiment, the data of the *in vitro* hemolysis and the overall hemolysis received by the input unit may be matched by using a respective patient identification (ID). The patient ID may be read by the input unit, e.g. with an optional barcode reading device (not shown) or be input manually.

In a preferred embodiment, the processing unit is further configured to correct the value of the at least one blood parameter data based on the calculated *in vitro* hemolysis data. The correction may occur as described above for the inventive method.

Optionally, the analysis apparatus further comprises a measurement unit 28 configured to determine the overall hemolysis in a whole blood sample of the patient that is suitable for determining the at least one blood parameter.

The input unit is configured to receive the overall hemolysis data from the measurement unit.

For example, the measurement unit 28 is provided as a porous mirror detection device, a chromatographic assay device, or a device capable of performing a visual hemolysis analysis, as described previously.

Optionally, the analysis apparatus 20 further comprises a measurement unit configured to determine the *in vivo* hemolysis in a fresh whole blood sample; wherein the input unit is configured to receive the *in vivo* hemolysis data from the measurement unit.

Optionally, the analysis apparatus 20 further comprises a device 30 configured to determine the at least one blood parameter in a whole blood sample. The measurement unit is further configured to determine the at least one blood parameter in a whole blood sample.

Optionally, the analysis apparatus 20 further comprises an output device 26. It is preferred that the output device 26 is configured to output the *in vitro* hemolysis data calculated by the processing unit 24 and/or the corrected value for the at least one blood parameter.

Optional examples of an output device 26 are a liquid crystal display (LCD), an organic light-emitting diode (OLED) display, and/or a printer. Furthermore, the output device 26 may comprise an output device driver capable of transmitting the *in vitro* hemolysis data over a LAN, WLAN, or a serial connection. Optionally, the output device 26 may comprise security features such as an encryption engine to ensure the security of patient data.

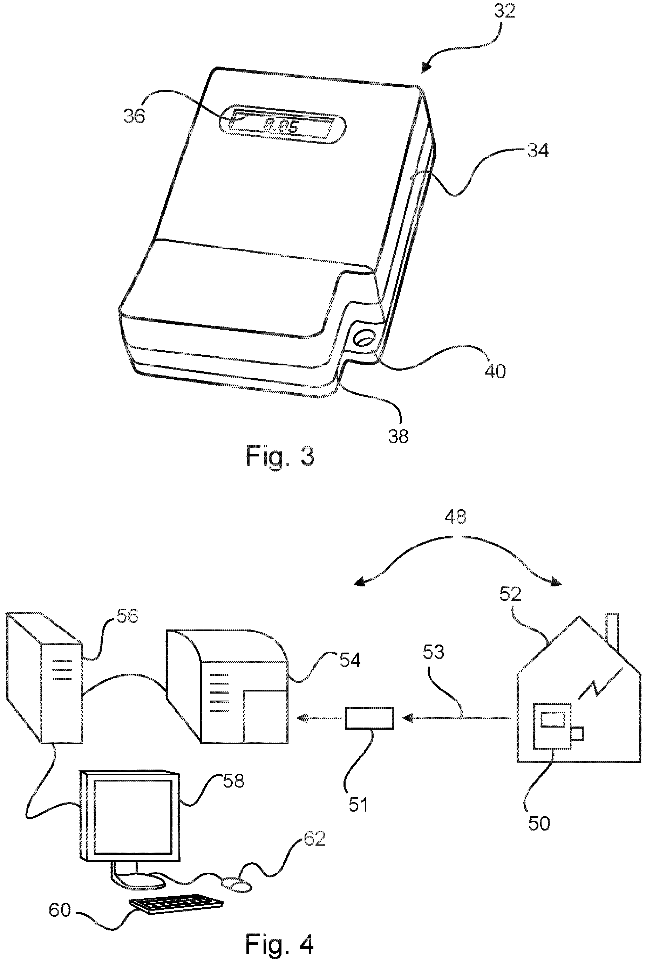

Figure 3 illustrates an example of a remote measurement unit 32 comprising a hand-holdable enclosure 34 (fabricated, for example, in ABS plastic) having a simple liquid crystal display 36 and a sample port 38. The sample port 38 is configured to receive a disposable sample carrier 40.

In operation, a fresh blood sample is obtained from a patient and deposited onto a new disposable sample carrier 40. The disposable sample carrier 40 is inserted into the sample port 38 of the remote measurement unit 32. The remote measurement unit 32 is initialised, either by means of a power switch actuated by the user, or automatically upon receipt of the disposable sample carrier 40 in the sample port 38. The remote measurement unit 32 then performs a test on the blood sample on the disposable sample carrier 40 to determine the *in vivo* hemolysis in the fresh blood sample.

The remote measurement unit 32 is optionally configured to display the *in vivo* hemolysis result to a user on the liquid crystal display 36, so that it may, for example, be noted, and transferred with a whole blood sample for further analysis.

In the simplest case, the *in vivo* hemolysis data may be read from the remote measurement device, and noted down by a patient or a medical professional and reported to the analysis device manually, for example, by post or over the telephone, or attached to a container containing a whole blood sample for transportation.

Optionally, the remote measurement unit 32 is configured to transfer *in vivo* hemolysis data via an (optionally encrypted) data connection to the analysis device. For example, the remote measurement device may optionally contain a GSM, CDMA, and/or LTE modem, or a WiFi and/or a LAN interface.

According to the third aspect, the present invention relates to a system 48 for analysing a whole blood sample, comprising:
- an analysis device 54 according to the second aspect or its optional embodiments described above; and
- a remote measurement unit 50 configured to determine *in vivo* hemolysis in a fresh blood sample, and to transmit the *in vivo* hemolysis data to the analysis device; wherein the input unit of the analysis device is configured to receive the *in vivo* hemolysis data determined and transmitted by the remote measurement unit. Figure 4 illustrates an example of a system 48 for analysing a whole blood sample.

The exemplary system comprises a remote measurement unit 50 in a patient's home, physician's practice, patient's hospital room or the like 52. Alternatively, the remote measurement unit 50 may be transported around by a medical professional or carer.

The system also comprises an analysis device 54 located in a medical laboratory. For the purpose of reading and formatting results, the analysis device 54 is, for example, connected to a personal computer 56 having a monitor 58, keyboard 60 and mouse 62.

In operation, a medical professional or carer or even the patient himself draws a whole blood sample 51 at the patient's home, physician's practice, patient's hospital room or the like 52, along with performing an *in vivo* hemolysis assessment using the remote measurement unit 50. The whole blood sample 51 is dispatched to the medical laboratory, and the *in vivo* hemolysis data is, in the illustrated example, transmitted to the medical laboratory via a label provided on the container of the whole blood sample 51. Optionally, the *in vivo* hemolysis data is provided as an (optionally encrypted) barcode or QR code, or in a USB stick. Alternatively, the *in vivo* hemolysis data may be transmitted via an (optionally encrypted) data link 53 provided, for example, over the internet, or a private FTP server hosted by the medical laboratory.

The *in vivo* hemolysis data is provided to the analysis device 54. The whole blood sample 51 is inserted into the analysis device 54. Given the *in vivo* hemolysis data, the analysis device 54 determines the overall hemolysis and optionally the at least one blood parameter in a whole blood sample of the patient that is suitable for determining the at least one blood parameter, and calculates 14 the *in vitro* hemolysis based on the overall hemolysis and *the in vivo* hemolysis. The analysis device 54 reports the results of the tests back to a medical professional using a personal computer 56, for example.

In a preferred embodiment, the system may further comprise a second remote measurement unit configured to determine the overall hemolysis and/or configured to determine the at least one blood parameter in a whole blood sample.

According to a fourth aspect, the present invention also relates to a computer program element for controlling an analysis apparatus of the present invention, which, when executed by a processing unit, cause said analysis apparatus to carry out the steps of the method of the present invention.

The computer program element may comprise, for example, a data structure containing computer instructions which, when executed by a processing unit of a computer, perform the calculation steps of the method of the first aspect.

According to a fifth aspect, the present invention also relates to a computer readable medium having stored the computer program element of the fourth aspect.

The invention may be embodied on a computer that has comprised the computer program element from the beginning, or a computer that has received, by means of a disc or Internet update the computer program element in the form of an update.

Optionally, the computer readable medium may comprise an optical storage or distribution medium, such as a CD-ROM disk, a DVD, or alternatively a solid-state storage or distribution medium such as a USB stick, or a magnetic disk.

Optionally, a program for providing the computer program element via downloading over the Internet is provided.

Although the invention has been illustrated and described with reference to the drawings and this description, these should be considered to be illustrative and exemplary, rather than restrictive. The invention is not limited to the disclosed embodiments. A skilled person studying the drawings, disclosure, and dependent claims can put into practice reasonable variations to the embodiments discussed herein. The reference signs are not to be construed as limiting the scope.

As used herein, the term "essentially the same time" means that the two whole blood samples were obtained from the patient within an amount of time in which it is highly unlikely that the value for the *in vivo* hemolysis might have changed.

As used herein, the term "anticoagulant" means a substance that prevents or reduces the coagulation of blood, i.e. the coagulation cascade leading to fibrin polymerization and therefore fibrin clot formation. Anticoagulants thereby prolong the clotting time by inhibiting the coagulation cascade by clotting factors after the initial platelet aggregation.

As used herein, the term "parameter" or "blood parameter" means any piece of clinical information about the blood sample. Preferably, blood parameters are the concentration of analytes present in the blood.

Where the term "comprising" is used in the present description and claims, it does not exclude other elements. For the purposes of the present invention, the term "consisting of" is considered to be a preferred embodiment of the term "comprising of".

Where an indefinite or definite article is used when referring to a singular noun, e.g. "a", "an" or "the", this includes a plural of that noun unless something else is specifically stated but however relates to a singular in a preferred embodiment.

As used herein, the term "at least one" as in "at least one blood parameter" means that only one type of agent or different agents, e.g. different blood parameters, may be present determined. In a preferred embodiment, "at least one" means "one" type of agent, e.g. one type of blood parameter, e.g. potassium.

As used herein, the term "blood sample" as in "whole blood sample" refers to a sample of blood that is suitable for diagnostic or analytical purposes. Thus, the blood sample comprises a relatively low volume of blood (from 20 µl to 10 ml blood), i.e. not the volumes e.g. required for blood donations (up to about 450 mL blood). A "blood sample that is suitable for determining at least one blood parameter" means that the blood sample is suitable for use in the determination of at least one blood parameter. For example, blood samples suitable for blood gas parameter and basic metabolic panel determination need to be prepared in a special blood sampler that ensures that the partial pressure of e.g. O₂ and CO₂ can be determined, e.g. by providing a special cap and/or removing air from the sampler filled with the blood sample.

As used herein, the term "whole blood" refers to blood composed of blood plasma and cellular components, e.g. erythrocytes, leucocytes and thromocytes. Preferably, the term "whole blood" refers to whole blood of a human subject or an animal subject, more preferably to whole blood of a human subject.

As used herein, the term "plasma" or "blood plasma" refers to the liquid part of the blood and lymphatic fluid that is devoid of cells.

As used herein, the term "translucent" refers to a material's property of allowing light to pass through.

As used herein, the term "blood sampler" means a device for collection of blood, such as a syringe, a capillary tube, or a test tube, e.g. an aspirating sampler or self-aspirating sampler, such as a Pico syringe (Radiometer Medical ApS), a vacuum test tube or a similar device designated for blood sampling.

As used herein, the term "fresh" as in a "fresh blood sample of the patient" refers to blood samples that are used for the analysis less than or equal to about 1 hour postdraw, preferably less than or equal to about 30 minutes postdraw, more preferably less than or equal to about 15 minutes postdraw, even more preferably less than or equal to about 10 minutes postdraw. Further, a fresh blood sample has not been exposed to conditions that may induce an *in vitro* hemolysis, such as a prolonged time of transport to the blood analysis, e.g. of more than 10 minutes, or vigorous mixing of the blood sample.

As used herein, the term "about" in the context of numeric values in the context of the present application, as e.g. in "about 10 minutes", means that the value recited immediately after the "about" also comprises minor deviations from the exact numeric value, e.g. due to measuring errors etc. In a preferred embodiment, the term "about" means a value within 15% (±15 %) of the value recited immediately after the term "about," including any numeric value within this range, the value equal to the upper limit (i.e., +15%) and the value equal to the lower limit (i.e., -15%) of this range. For example, the phrase "about 100" encompasses any numeric value that is between 85 and 115, including 85 and 115 (with the exception of "about 100%", which always has an upper limit of 100%). In one aspect, "about" means ±10 %, even more preferably ±5%, even more preferably ± 1% or less than ± 1%.

As used herein, the term "hemolysis" refers to the rupture of erythrocytes, e.g. due to chemical, thermal, mechanical or pathological influences, causing the release of hemoglobin and other internal components (e.g. potassium) of the erythrocytes into the surrounding fluid (e.g. blood plasma). Hemolysis may for example be determined by measuring the degree of free hemoglobin in a blood sample. The term "hemoglobin" refers to all naturally occurring forms of glycated and non-glycated hemoglobin and derivatives thereof. The term may refer to any molecule comprised of at least two globin subunits or domains of hemoglobin. Hemoglobin can be free in solution, i.e. "free hemoglobin", or contained within a cell, liposome or the like. However, the presence of hemolysis may also be measured by other methods detecting other analytes indicative of hemolysis, such as K⁺, LDH or carbonic anhydrase. In this context, it is understood that the analyte indicative of the hemolysis is of course not the same analyte as the at least one blood parameter. Thus, if K⁺ is the at least one blood parameter, the analyte indicative of hemolysis is for example the concentration of free hemoglobin.

As used herein, the term "overall hemolysis" refers to the level of hemolysis that can be measured in a blood sample without distinction whether the measured hemolysis might have occurred *in vivo* or *in vitro.*

As used herein, the term *"in vitro* hemolysis" refers to hemolysis that occurs due to improper specimen sample handling, such as an improper specimen sample collection, specimen sample processing or specimen sample transport. In particular, *in vitro* hemolysis may be caused by a high pressure drop and high shear or elongation rate, which may e.g. occur during filtration processes, when the sample is passed through a porous filter medium. Other important factors for *in vitro* hemolysis are the use of a catheter, intravenous or capillary blood collection; the needle gauge; the location of venipuncture; antiseptic used for phlebotomy; tourniquet time; traumatic draw; tube type; the collection tube being under filled; vigorous mixing; no mixing; syringe transfer or the specimen transport. Further factors for *in vitro* hemolysis include bacterial contamination, pressure, temperature, osmotic environment, pH value, contact with surfaces, frictional forces, or blood age and storage time of the unseparated whole blood sample.

As used herein, the term *"in vivo* hemolysis" refers to the rupture of red blood cells *in vivo.* In other words, the hemolysis did not occur *in vitro* due to the handling and processing of the blood sample. The *in vivo* hemolysis may itself be considered as a clinical condition and have more than 50 causes, including hereditary, acquired and iatrogenic conditions such as autoimmune hemolytic anemia and other hemoglobinopathies, HELLP (hemolysis, elevated liver enzymes and low platelets syndrome), transfusion reaction, or metabolic disorder such as liver diseases; chemical agents, e.g. drugs; physical agents, e.g. mechanical heart valves; or infectious agents, e.g. a bacterial infection. As used herein, terms such as "determining the *in vivo* hemolysis" do not refer to a medical method occurring *in vivo* but instead refer to the *in vitro* measurement of hemolysis in a blood sample in a diagnostic assay, wherein it can be assumed that the hemolysis already occurred *in vivo* and is not an artifact of the *in vitro* handling of the blood sample.

As used herein, the term "blood gas" as in "blood gas parameter" or in "blood gas analyzer" refers to gaseous parameters of the blood and includes the amounts of certain gases (e.g. oxygen and carbon dioxide) dissolved in blood, typically arterial blood. Blood gas parameters include the pH, pCO₂, pO₂, oxygen saturation (sO₂), the concentration of total hemoglobin (ctHb or tHb), the fraction of oxyhemoglobin (FO₂Hb or O₂Hb), the fraction of carboxyhemoglobin (FCOHb or COHb), the fraction of methemoglobin (FMetHb or MetHb), the fraction of deoxyhemoglobin (FHHb or RHb), and the fraction of fetalhemoglobin (FHbF). A "blood sample suitable for blood gas analysis" means that the blood sample may be used for measuring at least one blood gas parameter. Blood gas analyzer typically are also configured to measure additional other parameters, such as at least one basic metabolic panel (BMP) parameter.

As used herein, the term "basic metabolic panel" as in "basic metabolic panel parameter analysis" refers to biochemical blood parameters, in particular electrolytes, namely the concentration of Na⁺, K⁺, Cl⁻, HCO₃⁻, urea, creatinine, glucose (glu), Ca²⁺, lactate (lac) and total bilirubin (tBil).

### Examples

### Determining in vitro hemolysis and correcting the measured potassium concentration in a whole blood sample

Hemolysis was measured using a porous mirror sensor as described in the example of WO 2017/085180 A1.

Pooled donor whole blood samples were obtained and the porous mirror sensor was used to determine the *in vivo* hemolysis (H1).

For the determination of the overall hemolysis (H2), the occurrence of an *in vitro* hemolysis was simulated in the blood samples.

For a complete hemolysis sample, hemolysis was induced by freezing at -80°C for 30-40 minutes in a 4 mL aliquot of the pooled whole blood.

The protocol for preparing the completely hemolysed whole blood (100% HWB) was as follows:
1) One vacutainer tube was thoroughly homogenised by turning upside down 5-10 times.
2) 4 mL whole blood (WB) was pipetted into a Cryo-tube and put in the -80°C freezer for a minimum of 30 and a maximum of 40 minutes.
3) The frozen sample was brought to room temperature by letting the Cryo-tube rest for a minimum of 15 min. in a cup filled with water ( at room temperature).
4) The sample was transferred to a centrifuge tube.
5) The sample was spun at 1500 G for 15 minutes.
6) Approximately 80% of the supernatant was separated into a 30 mL cup using a 3 mL single-use pipette, leaving the hemolysed cell debris at the bottom of the centrifuge tube.
7) 0.5 mL were taken out with a syringe and the ctHb was measured on an ABL90 blood analyser (Radiometer Medical ApS).
8) The rest of the WB was pooled into a 100 mL beaker and homogenized by swirling the cup.
9) The sample was stirred gently with a 1 mL syringe before approx. 0.5 mL WB were taken out and aspirated into an ABL90 for measuring of the ctHb.

The level of desired overall hemolysis in a sample (H2) was prepared by mixing the 100% HWB and pooled un-hemolysed whole blood sample in a specified mixing ratio of 36.8 to 10,000. All pipetting is done using the reverse pipetting technique.

The preparation of the sample representing the overall hemolysis sample was performed as follows:
1) The calculated volume of 36.8 µL of 100% HWB were pipetted into a 100 mL cup for HWB 100.
2) The pooled WB was gently shaken until it was homogeneous and aspirated into the 10 mL pipette one time before actually metering the blood for the HWB-sample.
3) The calculated volume of 10 ml WB was added to the cup.
4) The HWB 100 solution was gently shaken in the cup before it was poured into a clean cup and back again to ensure sample homogeneity.
5) With a 10 mL pipette and reverse pipetting, three centrifuge tubes were filled with 9 mL HWB 100 each.
6) The tubes were centrifuged for 15 min. at 1500 G.
7) The rest of the WB sample was carefully drawn up into 10 mL syringes, carefully so as not to introduce extra hemolysis. The plunger was pressed so 2-3 mm air was left in the syringe to mix the sample.
8) The syringe with the hemolysed whole blood (HWB) sample rested at the table until approx. 15 minutes before the aspiration: It was put on the blood mixer, which rotated at 10 RPM, 15-60 minutes before the aspiration started.

The potassium level of samples before (TKC) and after hemolysis (UKC) were measured on the ABL90 analyser.

The results are summarized below in Table 2.

| | Abbreviations and formula | Sample |
|---|---|---|
| measured [K⁺] of *In vitro* hemolysed WB sample (µM) | UKC | 4089 |
| Hemolysis level measured in WB before hemolysis (mg Hb/dL); *"in vivo* hemolysis" | H1 | 7 |
| Hemolysis level measured in WB after *in vitro* hemolysis (mg Hb/dL); "overall hemolysis" | H2 | 115 |
| *In vitro* hemolysis (mg Hb/dL) | dH= H2-H1 | 108 |
| K⁺ concentration correction (µM) | KCC=α*dH | -324 |
| Corrected K⁺ concentration (µM) | CKC=UKC+KCC | 3765 |
| | | |
| True [K⁺] (µM) (measured in sample before hemolysis) | TKC | 3799 |
| | | |
| K⁺ Hemolysis error W.O. correction (µM) | UE=UKC-TKC | 290 |
| K⁺ Hemolysis error W. correction (µM) | CE=CKC-TKC | -34 |

The abbreviations used in Table 2 are described below:
- TKC: True K⁺ concentration
- UKC: Uncorrected K⁺ concentration - measured together with H2
- H1: First hemolysis measurement ("*in vivo* hemolysis")
- H2: Second hemolysis measurement ("overall hemolysis")
- dH: Difference in Hemolysis ("*in vitro* hemolysis")
- KCC: K⁺ concentration correction
- CKC: Corrected K concentration

- UE: Uncorrected error (error without *in vitro* hemolysis correction)
- CE: Corrected error (error with *in vitro* hemolysis correction)
- α: Conversion factor; -3 µM K⁺/(mg Hb/dL)

## Claims

1. An *in vitro* method for analyzing at least one blood parameter in a whole blood sample of a patient comprising:
a) determining (10) the *in vivo* hemolysis in a fresh whole blood sample of the patient by immediately performing the hemolysis determination after the blood sample has been obtained from the patient;
b) determining (12) the overall hemolysis and the at least one blood parameter in a whole blood sample of the patient; and
**characterized in that** the method is further comprising
c) calculating (14) the *in vitro* hemolysis based on the overall hemolysis and the *in vivo* hemolysis.

2. The method of claim 1 further comprising:
d) correcting the value for at least one parameter as determined in step b) based on the calculated *in vitro* hemolysis.

3. The method according to claim 1 or 2, wherein the at least one blood parameter is selected from the group consisting of potassium, calcium, lactate dehydrogenase, and sodium.

4. The method according to any one of claims 1 to 3, wherein the at least one blood parameter is potassium.

5. The method according to any one of claims 1 to 4, wherein step c) comprises performing a calculation comprising subtracting the value for the *in vivo* hemolysis from the overall hemolysis value.

6. The method according to any one of claims 2 to 5, wherein step d) comprises performing a calculation comprising subtracting a factor based on the *in vitro* hemolysis from the value of at least one additional blood parameter.

7. The method according to any one of claims 1 to 6, wherein the fresh whole blood sample of the patient and the whole blood sample of the patient for which the overall hemolysis and the at least one blood parameter were determined were created at essentially the same time.

8. An analysis apparatus (20) comprising:
- an input unit (22); and
- a processing unit (24);
wherein the input unit is configured to receive (i) *in vivo* hemolysis data of a fresh whole blood sample of a patient determined by immediately performing the hemolysis determination after the blood sample has been obtained from the patient, and (ii) overall hemolysis data and data of at least one blood parameter in a whole blood sample of the patient; and
**characterized in that** the processing unit is configured to calculate the *in vitro* hemolysis data based on the overall hemolysis data and the *in vivo* hemolysis data.

9. The analysis apparatus according to claim 8,
wherein the processing unit (24) is further configured to correct the value of the at least one blood parameter data based on the calculated *in vitro* hemolysis data.

10. The analysis apparatus according to any one of claims 8 or 9, further comprising a measurement unit (28, 32, 50) configured to determine the overall hemolysis in a whole blood sample of the patient;
wherein the input unit is configured to receive the overall hemolysis data from the measurement unit.

11. The analysis apparatus according to any one of claims 8 to 10, further comprising a device (30) configured to determine the at least one blood parameter in a whole blood sample; or wherein the measurement unit is further configured to determine the at least one blood parameter in a whole blood sample.

12. The analysis apparatus according to any one of claims 9 to 11, further comprising an output device (26);
wherein the output device is configured to output the presence of *in vitro* hemolysis and/or the corrected value for the at least one blood parameter.

13. A system for analysing a whole blood sample, comprising
- an analysis apparatus according to any one of claims 8 to 12;
- a remote measurement unit (50) configured to determine *in vivo* hemolysis in the fresh whole blood sample, and to transmit the *in vivo* hemolysis data to the analysis apparatus;
wherein the input unit of the analysis apparatus is configured to receive the *in vivo* hemolysis data determined and transmitted by the remote measurement unit, and wherein the system optionally further comprises a second remote measurement unit configured to determine the overall hemolysis and/or configured to determine the at least one blood parameter in a whole blood sample.

14. A computer program element for controlling an analysis apparatus (20) according to any one of claims 8 to 12, which, when executed by a processing unit, cause said analysis apparatus to carry out the steps of the method of any one of claims 1 to 7.

15. A computer readable medium having stored the computer program element of claim 14.

## Patentansprüche

1. In-vitro-Verfahren zur Analyse mindestens eines Blutparameters in einer Vollblutprobe eines Patienten, welches Folgendes umfasst:
a) Bestimmen (10) der In-vivo-Hämolyse in einer frischen Vollblutprobe des Patienten durch das sofortige Durchführen der Hämolysebestimmung, nachdem die Blutprobe von dem Patienten erhalten wurde;
b) Bestimmen (12) der Gesamthämolyse und des mindestens einen Blutparameters in einer Vollblutprobe des Patienten; und
**dadurch gekennzeichnet, dass** das Verfahren ferner Folgendes umfasst
c) Berechnen (14) der In-vitro-Hämolyse basierend auf der Gesamthämolyse und der In-vivo-Hämolyse.

2. Verfahren nach Anspruch 1, welches ferner Folgendes umfasst:
d) Korrigieren des Wertes für mindestens einen Parameter wie in Schritt b) bestimmt basierend auf der berechneten In-vitro-Hämolyse.

3. Verfahren nach Anspruch 1 oder 2, wobei der mindestens eine Blutparameter ausgewählt ist aus der Gruppe bestehend aus Kalium, Calcium, Laktatdehydrogenase und Natrium.

4. Verfahren nach einem der Ansprüche 1 bis 3, wobei der mindestens eine Blutparameter Kalium ist.

5. Verfahren nach einem der Ansprüche 1 bis 4, wobei Schritt c) das Durchführen einer Berechnung umfasst, welche das Subtrahieren des Wertes für die *In-vivo-*Hämolyse vom Gesamthämolysewert umfasst.

6. Verfahren nach einem der Ansprüche 2 bis 5, wobei Schritt d) das Durchführen einer Berechnung umfasst, welche das Subtrahieren eines Faktors basierend auf der *In-vitro-Hämolyse* vom Wert von mindestens einem zusätzlichen Blutparameter umfasst.

7. Verfahren nach einem der Ansprüche 1 bis 6, wobei die frische Vollblutprobe des Patienten und die Vollblutprobe des Patienten, für welche die Gesamthämolyse und der mindestens eine Blutparameter bestimmt wurden, im Wesentlichen zur gleichen Zeit gewonnen wurden.

8. Analysevorrichtung (20), welche Folgendes umfasst:
- eine Eingabeeinheit (22); und
- eine Verarbeitungseinheit (24);
wobei die Eingabeeinheit zum Empfangen von i) *In-vivo-*Hämolysedaten einer frischen Vollblutprobe eines Patienten, die durch sofortiges Durchführen der Hämolysebestimmung, nachdem die Blutprobe von dem Patienten erhalten wurde, bestimmt wurden, und von ii) Gesamthämolysedaten und Daten von mindestens einem Blutparameter in einer Vollblutprobe des Patienten konfiguriert ist; und
**dadurch gekennzeichnet, dass** die Verarbeitungseinheit zum Berechnen der In-vitro-Hämolysedaten basierend auf den Gesamthämolysedaten und den In-vivo-Hämolysedaten konfiguriert ist.

9. Analysevorrichtung nach Anspruch 8,
wobei die Verarbeitungseinheit (24) ferner zum Korrigieren des Wertes der mindestens einen Blutparameterdaten basierend auf den berechneten *In-*vitro-Hämolysedaten konfiguriert ist.

10. Analysevorrichtung nach einem der Ansprüche 8 oder 9, welche ferner eine Messeinheit (28, 32, 50) umfasst, die zum Bestimmen der Gesamthämolyse in einer Vollblutprobe des Patienten konfiguriert ist;
wobei die Eingabeeinheit zum Empfangen der Gesamthämolysedaten von der Messeinheit konfiguriert ist.

11. Analysevorrichtung nach einem der Ansprüche 8 bis 10, welche ferner ein Gerät (30) umfasst, das zum Bestimmen des mindestens einen Blutparameters in einer Vollblutprobe konfiguriert ist; oder wobei die Messeinheit ferner zum Bestimmen des mindestens einen Blutparameters in einer Vollblutprobe konfiguriert ist.

12. Analysevorrichtung nach einem der Ansprüche 9 bis 11, welche ferner ein Ausgabegerät (26) umfasst;
wobei das Ausgabegerät zum Ausgeben des Vorliegens von In-vitro-Hämolyse und/oder des korrigierten Wertes für den mindestens einen Blutparameter konfiguriert ist.

13. System zur Analyse einer Vollblutprobe, welches Folgendes umfasst:
- eine Analysevorrichtung nach einem der Ansprüche 8 bis 12;
- eine entfernte Messeinheit (50), die zum Bestimmen von *In-vivo*-Hämolyse in der frischen Vollblutprobe und zum Senden der In-vivo-Hämolysedaten an die Analysevorrichtung konfiguriert ist;
wobei die Eingabeeinheit der Analysevorrichtung zum Empfangen der In-vivo-Hämolysedaten, die durch die entfernte Messeinheit bestimmt und gesendet werden, konfiguriert ist und wobei das System wahlweise ferner eine zweite entfernte Messeinheit umfasst, die zum Bestimmen der Gesamthämolyse konfiguriert ist und/oder zum Bestimmen des mindestens einen Blutparameters in einer Vollblutprobe konfiguriert ist.

14. Computerprogrammelement zur Steuerung einer Analysevorrichtung (20) nach einem der Ansprüche 8 bis 12, welches, wenn es durch eine Verarbeitungseinheit ausgeführt wird, die Analysevorrichtung veranlasst, die Schritte des Verfahrens nach einem der Ansprüche 1 bis 7 auszuführen.

15. Computerlesbares Medium, auf dem das Computerprogrammelement von Anspruch 14 gespeichert ist.

## Revendications

1. Procédé *in vitro* d'analyse d'au moins un paramètre sanguin dans un échantillon de sang entier d'un patient comprenant les étapes consistant à :
a) déterminer (10) l'hémolyse *in vivo* dans un échantillon de sang entier frais du patient en effectuant immédiatement la détermination de l'hémolyse après que l'échantillon de sang a été obtenu du patient ;
b) déterminer (12) l'hémolyse globale et l'au moins un paramètre sanguin dans un échantillon de sang entier du patient ; et
**caractérisé en ce que** le procédé comprend en outre de
c) calculer (14) l'hémolyse *in vitro* sur la base de l'hémolyse globale et de l'hémolyse *in vivo.*

2. Procédé selon la revendication 1 comprenant en outre de :
d) corriger la valeur pour au moins un paramètre tel que déterminé dans l'étape b) sur la base de l'hémolyse *in vitro* calculée.

3. Procédé selon la revendication 1 ou 2, dans lequel l'au moins un paramètre sanguin est choisi dans le groupe constitué par le potassium, le calcium, la lactate déshydrogénase et le sodium.

4. Procédé selon l'une quelconque des revendications 1 à 3, dans lequel l'au moins un paramètre sanguin est le potassium.

5. Procédé selon l'une quelconque des revendications 1 à 4, dans lequel l'étape c) comprend la réalisation d'un calcul comprenant la soustraction de la valeur pour l'hémolyse *in vivo* de la valeur d'hémolyse globale.

6. Procédé selon l'une quelconque des revendications 2 à 5, dans lequel l'étape d) comprend la réalisation d'un calcul comprenant la soustraction d'un facteur basé sur l'hémolyse *in vitro* de la valeur d'au moins un paramètre sanguin supplémentaire.

7. Procédé selon l'une quelconque des revendications 1 à 6, dans lequel l'échantillon de sang entier frais du patient et l'échantillon de sang entier du patient pour lesquels l'hémolyse globale et l'au moins un paramètre sanguin ont été déterminés ont été créés essentiellement en même temps.

8. Appareil d'analyse (20) comprenant :
- une unité d'entrée (22) ; et
- une unité de traitement (24) ;
l'unité d'entrée étant configurée pour recevoir (i) les données d'hémolyse *in vivo* d'un échantillon de sang entier frais d'un patient déterminées en effectuant immédiatement la détermination de l'hémolyse après que l'échantillon de sang a été obtenu du patient et (ii) les données d'hémolyse globale et les données d'au moins un paramètre sanguin dans un échantillon de sang entier du patient ; et
**caractérisé en ce que** l'unité de traitement est configurée pour calculer les données d'hémolyse *in vitro* sur la base des données d'hémolyse globale et des données d'hémolyse *in vivo.*

9. Appareil d'analyse selon la revendication 8,
dans lequel l'unité de traitement (24) est en outre configurée pour corriger la valeur des données de l'au moins un paramètre sanguin sur la base des données d'hémolyse *in vitro* calculées.

10. Appareil d'analyse selon l'une quelconque des revendications 8 ou 9, comprenant en outre une unité de mesure (28, 32, 50) configurée pour déterminer l'hémolyse globale dans un échantillon de sang entier du patient ;
l'unité d'entrée étant configurée pour recevoir les données d'hémolyse globale de l'unité de mesure.

11. Appareil d'analyse selon l'une quelconque des revendications 8 à 10, comprenant en outre un dispositif (30) configuré pour déterminer l'au moins un paramètre sanguin dans un échantillon de sang entier ; ou l'unité de mesure étant en outre configurée pour déterminer l'au moins un paramètre sanguin dans un échantillon de sang entier.

12. Appareil d'analyse selon l'une quelconque des revendications 9 à 11, comprenant en outre un dispositif de sortie (26) ;
le dispositif de sortie étant configuré pour sortir la présence de l'hémolyse *in vitro* et/ou la valeur corrigée pour l'au moins un paramètre sanguin.

13. Système d'analyse d'un échantillon de sang entier, comprenant
- un appareil d'analyse selon l'une quelconque des revendications 8 à 12 ;
- une unité de mesure à distance (50) configurée pour déterminer l'hémolyse *in vivo* de l'échantillon de sang frais et pour transmettre les données d'hémolyse *in vivo* à l'appareil d'analyse ;
l'unité d'entrée de l'appareil d'analyse étant configurée pour recevoir les données d'hémolyse *in vivo* déterminées et transmises par l'unité de mesure à distance et le système comprenant en outre éventuellement une seconde unité de mesure à distance configurée pour déterminer l'hémolyse globale et/ou configurée pour déterminer l'au moins un paramètre sanguin dans un échantillon de sang entier.

14. Elément de programme informatique pour commander un appareil d'analyse (20) selon l'une quelconque des revendications 8 à 12, qui, quand il est exécuté par une unité de traitement, entraîne ledit appareil d'analyse à réaliser les étapes du procédé selon l'une quelconque des revendications 1 à 7.

15. Support lisible sur ordinateur ayant stocké l'élément de programme informatique selon la revendication 14.
